(19) **Europäisches Patentamt**

**European Patent Office**

**Office européen des brevets**

(11) **EP 0 862 406 B1**

(12) **EUROPÄISCHE PATENTSCHRIFT**

(45) Veröffentlichungstag und Bekanntmachung des Hinweises auf die Patenterteilung:
**18.02.2004 Patentblatt 2004/08**

(21) Anmeldenummer: **97940086.8**

(22) Anmeldetag: **16.08.1997**

(51) Int Cl.$^7$: **A61K 7/09**

(86) Internationale Anmeldenummer:
**PCT/EP1997/004487**

(87) Internationale Veröffentlichungsnummer:
**WO 1998/010735 (19.03.1998 Gazette 1998/11)**

(54) **MITTEL UND VERFAHREN ZUM DAUERHAFTEN VERFORMEN VON MENSCHLICHEN HAAREN**

AGENT AND PROCESS FOR PERMANENTLY SHAPING HUMAN HAIR

AGENT ET PROCEDE POUR PERMANENTER DES CHEVEUX NATURELS

(84) Benannte Vertragsstaaten:
**DE ES FR GB IT**

(30) Priorität: **14.09.1996 DE 19637495**

(43) Veröffentlichungstag der Anmeldung:
**09.09.1998 Patentblatt 1998/37**

(73) Patentinhaber: **Wella Aktiengesellschaft**
**64274 Darmstadt (DE)**

(72) Erfinder:
• **WEITZEL, Claudia**
**D-64291 Darmstadt (DE)**
• **MARESCH, Gerhard**
**D-64295 Darmstadt (DE)**
• **SENDELBACH, Gerhard**
**D-64297 Darmstadt (DE)**
• **LANG, Günther**
**D-64354 Reinheim (DE)**

(56) Entgegenhaltungen:
**EP-A- 0 075 475**     **EP-A- 0 091 740**
**EP-A- 0 636 358**     **EP-A- 0 689 827**
**US-A- 5 570 708**

EP 0 862 406 B1

## Beschreibung

Die vorliegende Erfindung betrifft ein Mittel und ein Verfahren zum schonenden dauerhaften Verformen von menschlichen Haaren im neutralen bis schwach alkalischen Bereich mit verbesserten Pflege- und Welleigenschaften.

Bekanntlich erfolgt die Dauerwellbehandlung menschlicher Haare in zwei Stufen: Die reduktive Spaltung der Cystin-Disulfidbrücken des Haares durch Einwirkung eines Reduktionsmittels und die anschließende Fixierung beziehungsweise Neutralisierung durch Aufbringung eines Oxidationsmittels, wodurch die Cystin-Disulfidbrücken wiederhergestellt werden.

Das klassische Reduktionsmittel ist dabei die Thioglykosäure, entweder als freie Säure oder in Form ihrer Salze, insbesondere des Ammoniumsalzes, wobei der pH-Wert dieser Zusammensetzung üblicherweise im alkalischen Bereich zwischen pH 8 und pH 10 liegt. Diese thioglykolathaltigen Zusammensetzungen können bei wiederholten, zeitlich nahe zusammenliegenden Anwendungen zu einer erhöhten Haarschädigung führen. Als weitere haarkeratinreduzierende Wirkstoffe können auch Sulfite. Thiomilchsäure, 3-Mercaptopropionsäure, Mercaptocarbonsäureester oder Cystein verwendet werden.

Die Fixierung beziehungsweise Neutralisierung des durch die Einwirkung von Thioverbindungen verformten Haares wird durch Einwirkung von Oxidationsmitteln, insbesondere Wasserstoffperoxid, vorgenommen.

Obwohl sich die genannten Haarverformungsmittel weiter Verbreitung erfreuen, besteht immer noch ein Bedürfnis, die Wellwirksamkeit und Pflegeeigenschaften, wie Griff und Kämmbarkeit solcher Zubereitungen, zu verbessern. Eine Möglichkeit zur Verbesserung der Pflegeeigenschaften besteht darin, die Reduktion der Haare bei niedrigerem pH-Wert durchzuführen (saure beziehungsweise Neutraldauerwelle) Dadurch werden Nebenreaktionen wie die Hydrolyse von Peptidbindungen, die zu erhöhter Haarschädigung führen, minimiert.

In der Vergangenheit hat man bereits versucht, diese Nachteile durch die Schaffung sogenannter "saurer Dauerwellmittel" zu überwinden, deren pH-Wert in der gebrauchsfertigen Form im Bereich von 6,8 bis 7,8 liegt.

Als Reduktionsmittel wurde in diesem pH-Bereich bisher Thioglykolsäureglycerinester verwendet. Wegen gelegentlich auftretender Sensibilisierungen will man jedoch auf den Einsatz dieser Verbindung verzichten. Eine andere Lösung besteht darin, höhere Mengen von Thioglykolsäure, gegebenenfalls in Kombination mit anderen Reduktionsmitteln, zu verwenden. Zur Erhöhung der Wellstärke muß jedoch Ammoniumcarbonat, Ammoniumhydrogencarbonat oder Ammoniumcarbamat zugesetzt werden. Derartige Zubereitungen sind aber unterhalb von pH 8 nicht lagerstabil, so daß eine Konfektionierung in 2-Komponentenverpackungen notwendig ist, bei dem die Komponenten erst unmittelbar vor der Anwendung zum gebrauchsfertigen Verformungsmittel zusammengegeben werden und das Mittel sodann kurzfristig aufgebraucht werden muß.

Es besteht deshalb ein Bedarf an sauren beziehungsweise pH neutralen Dauerverformungsmitteln in lagerstabiler Einkomponentenform, die somit auch die Konfektioformungsmitteln in lagerstabiler Einkomponentenform, die somit auch die Konfektionierung als Mehrportionspackung (mehrfache Entnahme möglich) gestatten.

In der EP - A- 0 629 395 wird empfohlen, Mittel zur Behandlung von Keratinfasem, die einen pH-Wert im Bereich von 2 bis 5 aufweisen sollen, mit organischen Säuren wie Glykolsäure einzustellen.

Es wurde nunmehr überraschend gefunden, daß einkomponentige, lagerstabile und ammoniumhydrogencarbonatfreie Dauerwellzubereitungen im pH-Bereich 5,1 bis 7,9, durch Zugabe von 0,1 bis 15 Gewichtsprozent Glykolsäure und/oder deren Salz erhalten werden, welche auch die übrigen vorstehenden Nachteile nicht aufweisen. Gegenstand der vorliegenden Erfindung ist dementsprechend ein einkomponentiges Mittel zur dauerhaften Verformung von Haaren vom pH 5,1 bis 7,9 mit einem Gehalt an einem haarkeratinreduzierenden Wirkstoff, ausgewählt aus Thioglykolsäure, Thioglykolsäureamiden, Thiomilchsäure, 3-Mercapto-propionsäure, Cystein, Cysteamin, Alkyl- oder Acylcysteaminen oder den Salzen dieser Verbindungen, dadurch gekennzeichnet, daß es 0,1 bis 15 Gewichtsprozent, vorzugsweise von 3 bis 10 Gewichtsprozent, Glykolsäure oder deren Salz enthält und frei von Ammoniumhydrogencarbonat ist.

Vorzugsweise enthält das erfindungsgemäße Mittel keinen Thioglykolsäureglycerinester. Die in dem Dauerverformungsmittel enthaltenen haarkeratinreduzierenden Wirkstoffe werden in dem Mittel zur dauerhaften Haarverformung bevorzugt in einer Menge von 2 bis 20 Gewichtsprozent, besonders bevorzugt in einer Menge von 4 bis 12 Gewichtsprozent und ganz besonders bevorzugt in einer Menge von 6 bis 10 Gewichtsprozent eingesetzt.

Neben den genannten oder anderen, an sich bekannten Reduktionsmitteln, enthalten sie üblicherweise ein Alkalisierungsmittel, dessen Konzentration von Art und Menge des haarkeratinreduzierenden Wirkstoffs abhängt. Die Dauerverformungsmittel enthalten als Alkalisierungsmittel bzw. als Mittel zur Einstellung des pH-Wertes insbesondere Ammoniak, Natronlauge oder wasserlösliche, physiologisch verträglichen organischen Basen, wie z.B. Di- oder Triethanolamin. Bevorzugtes Alkalisierungsmittel ist Ammoniak, wobei, je nach haaarkeratinreduzierendem Wirkstoff, die Einstellung des pH-Wertes auf den Bereich zwischen 5,1 und 7,9 angestrebt wird und dieser vorzugsweise im pH-Bereich von pH 6,8 bis 7,9 liegt.

Das Verformungsmittel kann sowohl in Form einer wäßrigen Lösung oder einer Emulsion als auch in verdickter Form auf wäßriger Basis, insbesondere als Creme, Gel oder Paste vorliegen.

Selbstverständlich kann das Verformungsmittel alle für derartige Mittel üblichen und bekannten Zusatzstoffe, zum Beispiel Verdickungsmittel, wie beispielsweise, Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quatemäre Ammoniumsalze, Alkylbetaine, oxethylierte Alkylphenole, Fettsäurealkanolamide oder oxethylierte Fettsäureester, femer Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, z. B. Polydimethyldiallylammoniumchlorid (CTFA Polyquaternium-6), Polydimethyl-aminoethylmethacrylat (zu 75% quaternisiert mit Diethylsulfat CTFA Polyquaternium-11), CTFA Polyquaternium-4, CTFA Polyquaternium-5, CTFA Polyquaternium-7, CTFA Polyquaternium-9, CTFA Polyquaternium-10, CTFA Polyquaternium-14, CTFA Polyquaternium-16, CTFA Polyquaternium-22, lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodigiykolsäure, Dithiomilchsäure, die Dithiole der genannten Verbindungen oder die jeweiligen Salze, zugesetzt werden.

Die vorliegende Erfindung betrifft weiterhin ein Verfahren zur dauerhaften Haarverformung. bei dem man die Haare bevor und/oder nachdem man sie in die gewünschte Form bringt, mit einem Verformungsmittel behandelt, mit Wasser spült. sodann oxidativ behandelt, mit Wasser spült, gegebenenfalls zur Wasserwelle legt und sodann trocknet, welches dadurch gekennzeichnet ist, daß als Verformungsmittel das vorstehend beschriebene erfindungsgemäße Mittel verwendet wird.

Bei einer bevorzugten Ausführungsform des erfindungsgemäßen Verfahrens wird das Haar zunächst mit einem Shampoo gewaschen und danach mit Wasser gespult. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignete Nachbehandlungsmittel verwendet werden. Beispiele für in solchen Nachbehandlungsmitteln verwendbare Oxidationsmittel sind Kalium- und Natriumbromat. Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel. Pflegestoffe wie kationische Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserweite gelegt und schließlich getrocknet.

Darüber hinaus hat sich gezeigt, daß sich durch Zusatz von Glykolsäure beziehungsweise deren Salz, insbesondere deren Ammoniumsalz, eine Verbesserung der Kämmbarkeit und des Griffes des Haares erzielen läßt. Zusätzlich wurde überraschender Weise festgestellt, daß sich mit zunehmendem Gehalt an Glykolsäure die Wellwirksamkeit beziehungsweise die Wellstabilität deutlich erhöht.

Zum Vergleich der Wellwirksamkeit wurden an blondierten Haarsträhnen aus mitteleuropäischem Haar Wellstabilitäten ermittelt. Hierzu wurden 16,5 cm lange, aus etwa 100 Haaren bestehende Strähnchen auf Wickler mit einem Innendurchmesser von drei Millimetern gewickelt und mit den Dauerwellösungen im Verhältnis 1 : 1,2 bei 45°C und einer Einwirkungszeit von 10 Minuten behandelt. Anschließend wurde das Haar mit einer peroxidhaltigen Fixierung fixiert, getrocknet und während vier Stunden Aushängung in Wasser die Weltstabilität bestimmt.

Die Wellstabilität errechnet sich gemäß der Formel:

$$\text{Wellstabilität [\%]} \ \frac{l_0 - l_t}{l_0 - l_1} \times 100$$

$l_0$ = Gesamtlänge der nicht umgeformten, gestreckten Strähne (165 mm)
$l_t$ = Länge der abgewickelten, ausgehängten Strähne nach 240 Minuten
$l_1$ = Länge der aufgewickelten Strahne

Der verwendete Wickler hat einen Durchmesser von 3 mm und eine Länge von 35 mm ($l_1$ ist dementsprechend 35 mm )

|  | Wellstabilität nach 15 Minuten Einwirkungszeit |
|---|---|
| Standard | 47,9 % |
| Standard + 5,0 Gew. % Glykolsäure | 51,4 % |
| Standard + 10,0 Gew.% Giykolsäure | 56,0 % |

Standard:    13,0 % Ammoniumthioglykolat 70 %ig, mit Ammoniak 25 %ig auf pH 7,5 eingestellt, ad 100 Wasser, vollentsalzt

Die nachfolgenden Ausführungsbeispiele dienen der näheren Illustration der Erfindung

**Beispiel 1**

| 13,0 % | Ammoniumthioglykolat, 70%ig |
|---|---|
| 5,0 % | Ammoniumglykolat |
| 0,5 % | kationisches Polymer CTFA POLYQUATERNIUM-6 mit Ammoniak auf pH 7,3 einstellen |
| ad 100,0% | Wasser, vollentsalzt |

Durch Farbbehandlungen vorgeschädigtes Haar wird mit einem Shampoo gewaschen, frottiert und auf Wickler mit einem Durchmesser von 8 mm gewickelt. Anschließend wird das Behandlungsmittel oben genannter Rezeptur gleichmäßig auf dem gewickelten Haar verteilt. Nach einer Einwirkungszeit von 10 Minuten bei einer Temperatur von 40°C wird das Haar mit Wasser gespult und mit 100 g einer 2,5 %igen wäßrigen Wasserstoffperoxidlösung oxidativ nachbehandelt. Nach Entfernung der Wickler werden die Haare erneut mit Wasser gespült und anschließend getrocknet. Als Ergebnis dieser Behandlung zeigte sich eine gleichmäßig, elastische Verformung der Haare mit deutlich verbesserten Pflegekriterien hinsichtlich Griff und Kämmbarkeit im feuchten Haar

**Beispiel 2**

| 17,0 % | Ammoniumthioglykolat, 70 %ig |
| 2,0 % | Thiomilchsäure |
| 4,0 % | Ammoniumglykolat |
| 0,3 % | Copolymerisat aus Vinylpyrrolidon/Dimethylaminoethylmethacrylat (80:20) (CTFA POLYQUATERNIUM-11), |
| 4,0 % | Harnstoff |
| 0,8 % | Cocobetain |
| 0,2 % | Parfümöl, Trübungsmittel |
| | mit Ammoniak. 25 %ig auf pH 7,5 einstellen |
| ad 100 % | Wasser, vollentsalzt |

Bei normalen nicht vorgeschädigten Haaren ließen sich ebenfalls eine gleichmäßige und lebhafte Umformung sowie sehl gute Pfleoeeigenschaften erzielen wie im oben genanntes Beispiel.

**Patentansprüche**

1. Einkomponentiges Mittel zur dauerhaften Verformung von Haaren vom pH 5,1 bis 7,9 mit einem Gehalt an einem haarkeratinreduzierenden Wirkstoff, ausgewählt aus Thioglykolsäure, Thioglykolsäureamiden, Thiomilchsäure, 3-Mercapto-propionsäure, Cystein, Cysteamin, Alkyl- oder Acylcysteaminen oder den Salzen dieser Verbindungen, **dadurch gekennzeichnet, dass** es 0,1 bis 15 Gewichtsprozent Glykolsäure oder deren Salz enthält und frei von Ammoniumhydrogencarbonat ist.

2. Mittel nach Anspruch 1, **dadurch gekennzeichnet, dass** es als alleinigen haarkeratinreduzierenden Wirkstoff Thioglykolsäure enthält.

3. Mittel nach einem der Ansprüche 1 bis 2, **dadurch gekennzeichnet, dass** die Glykolsäure oder deren Satz in einer Menge von 3 bis 10 Gewichtsprozent enthalten ist.

4. Mittel nach einem der Ansprüche 1 bis 3, **dadurch gekennzeichnet, dass** der pH-Wert des Mittels 6,8 bis 7,9 beträgt.

5. Mittel nach einem der Ansprüche 1 bis 4, **dadurch gekennzeichnet, dass** es ein kationisches Polymer enthält.

6. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, dass** man als Verformungsmittel ein Mittel nach einem der Ansprüche 1 bis 4 verwendet.

7. Verfahren nach Anspruch 6, **dadurch gekennzeichnet, dass** man das Verformungsmittel 5 bis 30 Minuten lang einwirken lässt.

8. Verfahren nach Anspruch 6, **dadurch gekennzeichnet, dass** man das Verformungsmittel unter Anwendung von Wärme 5 bis 20 Minuten lang einwirken lässt.

**Claims**

1. Single-component agent for the permanent shaping of hair of pH 5.1 to 7.9 with a content of a hair keratin-reducing active ingredient chosen from thioglycolic acid, thioglycolamides, thiolactic acid, 3-mercaptopropionic acid, cysteine, cysteamine, alkyl- or acylcysteamines or the salts of these compounds, **characterized in that** it comprises 0.1 to 15 per cent by weight of glycolic acid or salt thereof and is free from ammonium hydrogencarbonate.

**2.** Agent according to Claim 1, **characterized in that** it comprises thioglycolic acid as the sole hair keratin-reducing active ingredient.

**3.** Agent according to one of Claims 1 to 2, **characterized in that** the glycolic acid or salt thereof is present in an amount of from 3 to 10 per cent by weight.

**4.** Agent according to one of Claims 1 to 3, **characterized in that** the pH of the agent is 6.8 to 7.9.

**5.** Agent according to one of Claims 1 to 4, **characterized in that** it comprises a cationic polymer.

**6.** Method of permanently shaping hair in which the hair, before and/or after it is held in the desired shape, is treated with a shaping agent, rinsed with water, oxidatively after-treated, rinsed again with water, optionally placed in a water-wave and then dried, **characterized in that** the shaping agent used is an agent according to one of Claims 1 to 4.

**7.** Method according to Claim 6, **characterized in that** the shaping agent is left to act for 5 to 30 minutes.

**8.** Method according to Claim 6, **characterized in that** the shaping agent is left to act under the application of heat for 5 to 20 minutes.

**Revendications**

**1.** Composition à un seul composant pour la mise en forme permanente des cheveux de pH 5,1 à 7,9, ayant une teneur en une substance active réduisant la kératine des cheveux, choisie parmi l'acide thioglycolique, les thioglycolamides, l'acide thiolactique, l'acide 3-mercaptopropionique, la cystéine, la cystéamine, les alkyl- ou acylcystéamines et les sels de ces composés, **caractérisée en ce qu'**elle contient de 0,1 à 15 % en poids d'acide glycolique ou d'un sel de celui-ci et est exempte d'hydrogénocarbonate d'ammonium.

**2.** Composition selon la revendication 1, **caractérisée en ce qu'**elle contient en tant que seule substance active réduisant la kératine des cheveux de l'acide thioglycolique.

**3.** Composition selon la revendication 1 ou 2, **caractérisée en ce que** l'acide glycolique ou un sel de celui-ci est contenu en une quantité de 3 à 10 % en poids.

**4.** Composition selon l'une quelconque des revendications 1 à 3, **caractérisée en ce que** le pH de la composition est de 6,8 à 7,9.

**5.** Composition selon l'une quelconque des revendications 1 à 4, **caractérisée en ce qu'**elle contient un polymère cationique.

**6.** Procédé pour la mise en forme permanente des cheveux, dans lequel les cheveux, avant et/ou après avoir été maintenus sous la forme désirée, sont traités par une composition de mise en forme, rincés à l'eau, soumis à un après-traitement par oxydation, rincés à nouveau à l'eau, éventuellement mis en plis et ensuite séchés, **caractérisé en ce qu'**on utilise comme composition de mise en forme une composition selon l'une quelconque des revendications 1 à 4.

**7.** Procédé selon la revendication 6, **caractérisé en ce qu'**on laisse agir la composition de mise en forme pendant 5 à 30 minutes.

**8.** Procédé selon la revendication 6, **caractérisé en ce qu'**on laisse agir la composition de mise en forme, avec application de chaleur, pendant 5 à 20 minutes.